# EUROPÄISCHE PATENTSCHRIFT

(11) **EP 0 390 883 B1**
(45) Veröffentlichungstag und Bekanntmachung des Hinweises auf die Patenterteilung: **07.12.1994**
(21) Anmeldenummer: 89909735.6
(22) Anmeldetag: 11.09.1989
(51) Int. Cl.: A61F 2/36, A61F 2/46

(54) **HÜFTGELENKSPROTHESE UND IHRE VERWENDUNG**
HIP-JOINT PROSTHESIS AND USE THEREOF
PROTHESE DE LA HANCHE ET SON UTILISATION

(30) Priorität: 09.09.1988 DE 3830748
(43) Veröffentlichungstag der Anmeldung: 10.10.1990
(60) Teilanmeldung: 94107864.4
(73) Patentinhaber: DRAENERT, Klaus, Dr.med., D-81545 München (DE)
(72) Erfinder: DRAENERT, Klaus, Dr.med., D-81545 München (DE)
(74) Vertreter: VOSSIUS & PARTNER
(86) Internationale Anmeldenummer: EP8901054
(87) Internationale Veröffentlichungsnummer: WO9002533

(56) Entgegenhaltungen:
- EP-A- 0 065 481
- EP-A- 0 102 853
- EP-A- 0 128 036
- EP-A- 0 145 641
- EP-A- 0 201 407
- EP-A- 0 257 222
- DE-U- 8 705 921
- FR-A- 2 104 009
- FR-A- 2 438 469
- US-A- 4 808 186

## Beschreibung

Die Erfindung betrifft eine Hüftgelenksprothese, die vorzugsweise mit Knochenzement, aber auch zementfrei eingesetzt werden kann.

In der orthopädischen Chirurgie sind Gelenkersatzoperationen Standardverfahren der Therapie geworden, gleichwohl können die bisher zum Einsatz kommenden Prothesen und ihre Verankerungsverfahren noch nicht als Lösung aller bei den Operationen auftretender Probleme angesehen werden. So kommt es beispielsweise bei zementierten Gelenkersatzkomponenten sehr oft zur Zerstörung des Zementköchers und zur Auslockerung der Prothese. Es wurden daraufhin eine Reihe von Prothesen entwickelt, die ohne Verwendung von Knochenzement implantiert werden können. Die bekannten zementfrei implantierten Prothesen weisen jedoch ebenfalls noch Nachteile auf. Beispielsweise konnte festgestellt werden, daß zementfreie Implantate in der ersten Zeit nach der Operation Schmerzen verursachen und in der nachfolgenden Zeit ziemlich unkontrollierte Atrophien am Knochen hervorrufen, die zu pathologischen Frakturen Anlaß geben können (s. I.W Brown und P.A. Ring, Osteolytic changes in the upper femoral shaft following porous-coated hip replacement, J. Bone Joint Surg. 67B, S. 218-221). Darüber hinaus hat die Zahl der Lockerungen durch die zementfreien Prothesen keinesfalls abgenommen, sondern zugenommen.

Im Rahmen von Versuchen konnte am Beispiel des Hüftgelenkes und hier speziell an der Femurkomponente gezeigt werden, daß die unzementierten Komponenten eine erhebliche Deformation des Knochens in den Belastungsphasen verursachen. Diese Deformation ist umso größer, je mehr Masse ein Implantat mit sich bringt, bzw. je größer die Deformierbarkeit des individuellen Knochens ist. Auf der anderen Seite ist es für die zementfreien Implantate zwingend, von Beginn an einen schlüssigen Kontakt zum Knochen zu gewährleisten, was zur Entwicklung von formschlüssigen oder anatomisch adaptierten Komponenten geführt hat (s. J. Henssge, Methode zur Entwicklung anatomisch richtiger Implantatkörper, in "Grenzschichtprobleme der Verankerung von Implantaten unter besonderer Berücksichtigung von Endoprothesen", herausgegeben von M. Jäger, M.H. Hackenbroch, H.J. Refior, Georg Thieme Verlag Stuttgart, New York, 1981). Ein Extrem dieser Entwicklung ist die sogenannte costum made-Prothese, die beispielsweise von Mulier vorgestellt worden ist (J.C. Mulier, Improvements of the Charnely technique of total hip replacement and future developments, Acta Orthop. Belg. 52, Seiten 392 bis 403). Diese Designs führen aber zu besonders schweren Implantaten. Die Reaktion des Knochens ließ dementsprechend eine kräftige Deformation erkennen. Deformationen des Knochens führen aber zu Oberflächenverschiebungen und damit zur Knochenresorption, was die Auslockerung der Prothese zur Folge haben kann.

Es wurden auch Versuche unternommen, die Erfahrungen mit anderen Implantaten auf die Prothetik zu übertragen. So wurde beispielsweise das Prinzip der Vorspannung, welches ein Grundprinzip der Osteosynthesetechnik darstellt, mit dem sogenannten press-fit-Prinzip in die Endoprothetik eingebracht (M.E. Müller, Total hip replacement: planning, technique and complications, in: Cruess, R.L. Mitchell, N.S.: Surgical management of degenerative arthritis of the lower limb, Chapter 10,Seiten 91 bis 113, Lea & Febiger, Philadelphia 1975; M.E. Müller und B. Elmiger: Coxarthrose, 10-Jahres-Ergebnisse der sog. Setzholz-Totalprothese, Orthopäde 8, Seiten 73 - 74, 1979; K. Zweymüller, Knochen- und Gelenkersatz mit biokeramischen Endoprothesen, Facultas, Wien, 1978). Auch bei Anwendung des Prinzips der Vorspannung war jedoch wie bei den schweren Prothesenkomponenten eine starke Deformation die hervorstechende Antwort des Lagerknochens.

Die Untersuchung mehr oder weniger gut zementierter Prothesen, welche lange Jahre getragen worden waren, ließ die Überlegenheit zementierter Komponenten erkennen. Ein geringes Remodelling, d.h. ein geringer Umbau des knöchernen Lagers, war Hinweis für eine gleichmäßige Krafteinleitung, und der geschlossene Knochenkontakt war der Beweis für das Fehlen von Relativbewegungen im Interface, was auf eine relativ geringe Deformation des Knochens durch das Implantat schließen läßt (K. Draenert, Histomorphologische Befunde zur gedämpften und ungedämpften Krafteinleitung in das knöcherne Lager, Vereinigung Nordwestdeutscher Orthopäden, 36. Jahrestagung, Hannover, 15. bis 18. Juni 1986).

Der Erfindung liegt somit die Aufgabe zugrunde, eine Prothese (Endoprothese) bzw. ein Implantat zu entwickeln, welches reproduzierbar gute Ergebnisse im Bezug auf die Einheilung und Beanspruchbarkeit erreichen läßt.

Zur Lösung dieser Aufgabe geht die Erfindung von dem Grundgedanken aus, bei der Konstruktion der Prothese die Zusammenhänge zwischen Deformation und Masse des Implantats bzw. der Deformierbarkeit des Knochens zu berücksichtigen. Es konnte im Rahmen der Erfindung gezeigt werden, daß nicht nur die Dämpfung der Krafteinleitung zu einer geringeren Deformation des Knochens führt, sondern daß die Deformation des Knochens sich auch ganz erheblich von Individuum zu Individuum unterscheidet bzw. beeinflußt werden kann, beispielsweise durch die Gestaltung der Prothese und durch Aussteifung mit Knochenzement.

Die vorstehende Aufgabe wird durch die Prothese gemäß der Erfindung gelöst. Die erfindungsgemäße Prothese berücksichtigt und löst die Probleme der Deformation des Knochens durch das Implantat.

Gegenstand der Erfindung ist ferner die Anwendung der erfindungsgemäßen Hüftgelenksprothese bei Hüftgelenksoperationen.

Die erfindungsgemäße Prothese zeichnet sich dadurch aus, daß sie die größtmögliche Oberfläche, die für die Krafteinleitung in die Prothese bzw. in den Knochen zur Verfügung steht, ausnützt und ein Höchstmaß an Rotationsstabilität aufweist. Die Prothese kann mit ihrer Masse der individuellen Deformierbarkeit des Knochens angepaßt werden und kann im Extremfall als Leichtbaukonstruktion ausgeführt werden.

Die erfindungsgemäße Prothese läßt sich derart einsetzen, daß nur ein geringer Knochenverlust entsteht. Beim Verfahren zum Einsetzen der Prothese wird zunächst mit Diamanthohlschleifen ein Schlitz gefräst. Die Zementauffüllung erfolgt ebenfalls über mit Diamanthohlschleifen gesetzte Verankerungsstollen unter Beaufschlagung mit einem starken Vakuum. Bei zementfreier Implantation kann die erfindungsgemäße Prothese aufgrund ihrer Konstruktion vorgespannt werden und so porösen oder mit Keramik beschichteten Implantaten für die Zeit der Knochenheilung eine mechanisch stabile Fixation gewährleisten.

Die erfindungsgemäße Prothese weist einen seitlich (lateral) offenen Hohlkörper auf, der sich den kraftübertragenden Flächen anlegt, und stellt ein offenes Hohlkörperimplantat dar. Vorzugsweise nimmt die Steifigkeit der Prothese von proximal nach distal ab.

Hohlkörperimplantate als solche sind grundsätzlich bereits bekannt. So ist aus der EP-A-0190446 ein metallenes Knochenimplantat bekannt, das aus zwei zu einem geschlossenen Hohlkörper zusammengefügten Blechschalen besteht. Durch die Ausbildung als geschlossener Hohlkörper wird zwar das Gewicht des Implantats reduziert; die Steifigkeit des Implantats entspricht aber im wesentlichen herkömmlichen Vollimplantaten und die vorstehend erläuterten Probleme im Zusammenhang mit der Kraftübertragung und der Deformierbarkeit des Knochens werden hierdurch nicht gelöst.

Aus der EP-A-0065481 ist eine Hüftgelenksprothese mit einem Hohlschaft bekannt, der durch lochförmige Aussparungen örtliche Materialschwächungen aufweist, durch die der Hohlschaft bezüglich seiner Längs- und/oder Biegesteifigkeit an die Längs- bzw. Biegesteifigkeit des umgebenden Knochenbereichs angepaßt werden soll. Auch dieses Implantat vermag die vorstehend erläuterten Probleme nicht zu lösen.

Aus FR-A-2 438 469 ist eine Hüftgelenksprothese mit einem Schaft in Form eines im wesentlichen rechteckigen Rohres bekannt, dessen eine kurze Seite praktisch vollständig entfernt ist, so daß der Schaft einen im wesentlichen C-förmigen Querschnitt aufweist. Die offene Seite des Schaftes ist an der medialen Seite der Prothese angeordnet, wo nach den der vorliegenden Erfindung zugrundeliegenden Erkenntnissen die hauptsächliche Kraftübertragung zwischen Knochen und Prothese stattfindet, so daß die Prothese gemäß FR-A-2 438 469 kein großes Interface zur Kraftübertragung zwischen der Prothese und dem Knochen zur Verfügung stellen kann.

Ferner ist eine zementfreie, selbstverblockende Hüftgelenkprothese bekannt, bei der aus dem vollen Prothesenschaft Löcher ausgeschnitten sind, so daß lediglich dünne Verbindungsstege verbleiben, die den medialen und lateralen Teil der Prothese verbinden (S. Tepic, S.M. Perren, Cementless self-locking stem for hip prosthesis).

Demgegenüber weist die erfindungsgemäße Prothese in einer Ausführungsform seitlich entlang ihres Stiels und gegebenenfalls ihres Halsteils eine durchgehende Öffnung oder Rinne in Form eines langgezogenen Schlitzes auf, der sich über den wesentlichen Teil der Länge des Implantats, vorzugsweise über mehr als 80 % oder gegebenenfalls über mehr als 90 % der Länge des Implantats erstreckt. In diesem Bereich weist die Prothese einen U-förmigen oder hufeisenförmigen Querschnitt auf, wobei der Steg des U bzw. des Hufeisens den medialen Teil der Prothese bildet und die Enden der Arme des U abgerundet sind. Die Prothese ist dort offen, wo keine oder nur geringe Kraftübertragung erfolgen muß. Die Prothese stellt deshalb die größtmögliche Oberfläche für die Kraftübertragung bei gleichzeitiger Verringerung der Masse zur Verfügung und kann in verschiedener Weise den Bedürfnissen des Patienten angepaßt werden. Die Tiefe der Rinne kann bis zu 90 % der Ausdehnung der Prothese in ihrer Medial-Lateral-Richtung betragen, vorzugsweise etwa 30 bis 80 %, besonders bevorzugt etwa 40 bis 70 %, und kann in Längsrichtung der Prothese variieren.

Die erfindungsgemäße Prothese weist vorzugsweise einen Kopfteil, einen Halsteil und den Stiel oder Schaft auf.

Der Kopfteil ist vorzugsweise als Halbschale ausgebildet, welche lediglich das tragende Dach des Femurkopfes bedeckt, da nur dort eine Kraftübertragung erfolgt. Um der Schreitbewegung Rechnung zu tragen, ist vorzugsweise vorne (ventral) und hinten (dorsal) die Gleitfläche tiefer heruntergezogen.

Der Kopfteil kann auch als Vollersatz ausgebildet sein, der einem Konus im Halsteil aufgesetzt wird oder selbst mit einem Konus versehen ist und in den Halsteil eingesteckt werden kann. Der Kopfersatz kann auch einem ausgezogenen und vorzugsweise massiv ausgebildeten Plateau des Halsteiles direkt aufgeschweißt oder aufgesteckt werden.

Der Halsteil der Prothese ist massiver und gegebenenfalls als Vollkörper ausgebildet und stellt den Übergang zur im Querschnitt vorzugsweise U-förmigen Rinne des Stieles dar, dient aber auch gegebenenfalls zur Aufnahme eines Gelenkes, als Gleitschiene oder als Steckgelenk, wenn eine modulare Implantatkomponente eingesetzt werden soll. Bei dieser Ausführungsform ist es auch möglich, die Halbschale der tragenden Fläche gegen einen konventionellen Steckkonus auszutauschen. Sofern noch ein Teil des Knochens vom Schenkelhalskopf erhalten ist, ist es auch möglich, einen entsprechend modifizierten Kopf aufzuschieben, wodurch eine wesentlich größere kraftaufnehmende Fläche am Schenkelhals erhalten bleibt. Die Prothese kann aber auch einteilig aus einem Stück hergestellt sein; der Halsteil liegt dann an den starken Strukturen des Schenkelhalses lateral auf.

Bei einer Ausführungsform der Erfindung ist der Kopf oder der den Kopf aufnehmende Konus am Halsteil ein-, zwei- oder dreidimensional einstellbar oder verschiebbar angeordnet. Beispielsweise kann ein auf eine Steckschiene aufgesteckter Kopf in der Frontalebene und/oder in der Sagittalebene verstellt werden. Auf diese Weise gelingt es, für verschiedene Konfigurationen von Patienten exakt ein Alignment einzustellen. Unter Alignment versteht man die exakte Ausrichtung des Kopfes in Richtung auf und die Einleitung der Kraft in das Sacroiliacalgelenk, das physiologischerweise die Kraft aufnimmt und auf die Wirbelsäule überträgt. Vorzugsweise ist die Einstelleinrichtung so ausgebildet, daß die Einstellbarkeit in den einzelnen Richtungen unabhängig voneinander gegeben ist, um eine möglichst große Freiheit und Anpaßbarkeit beim Alignment zu gewährleisten.

Der Kopf kann in jeder beliebigen Stellung, beispielsweise durch Stellschrauben fixiert werden. Der Halsteil kann ebenso in der Länge verstellt werden, wodurch eine exakte Einstellung in die Belastungsebene, beispielsweise aus Trochantermittelpunkt, Rotationszentrum, Symphysenzentrum erreicht wird.

Der Stiel ist in seinem Querschnitt vorzugsweise als offenes Halbrohr oder entlang seines Umfangs zu etwa der Hälfte bis 3/4 geschlossenes Rohr, vorzugsweise als zu 2/3 bis 3/4 geschlossenes Rohr ausgebildet, das zur Prothesenspitze hin konisch zuläuft. Es ist wesentlich, daß der Stiel exakt entlang der durch Verlängerung der freien Markhöhle gebildeten Achse lateral eingeführt wird, ohne daß dadurch die Schenkelhalsstrukturen unnötigerweise verletzt werden. Das Halbrohr oder die Hohlrinne legt sich mit ihrem im Querschnitt konvexen Teil der starken Knochenstruktur des proximalen Femurquerschnittes an.

Bei einer Ausführungsform der Erfindung ist die Massenverteilung in jeder Querschnittsfläche zumindest im proximalen Abschnitt so gestaltet, daß die Hauptmasse dorsal und medial ausgerichtet ist. Dies beruht darauf, daß systematische Untersuchungen anhand von Röntgenbildern gezeigt haben, daß in diesen Bereichen die hauptsächliche Kraftübertragung erfolgt. Wegen dieser Asymmetrie ist in der Regel eine Rechtsversion und eine Linksversion der Prothese erforderlich.

In Richtung auf das distale Ende der Prothese nimmt die mediale Ausrichtung der Masse allmählich ab, und in der distalen Prothesenhälfte ist die mediale und laterale Massenverteilung bezüglich der Achse im wesentlichen symmetrisch. Die dorsale Ausrichtung der Prothesenmasse nimmt in Richtung auf das distale Ende der Prothese rasch ab, und in der distalen Prothesenhälfte ist die Masse vorwiegend ventral ausgerichtet. In der Seitenansicht betrachtet ist die dorsal-ventrale Massenverteilung im wesentlichen S-förmig ausgebildet: der im proximalen Bereich vorliegende dorsale Massenüberschuß geht im mittleren Prothesenabschnitt gegen Null und wird im distalen Prothesenabschnitt negativ, d.h. es liegt ein ventraler Massenüberschuß vor, der an der Prothesenspitze wieder zu Null wird. Dennoch ist die Prothese als Geradschaftprothese zu bezeichnen, da die beispielsweise zwischen dem Einschlagpunkt der Prothese und der Prothesenspitze verlaufende Prothesenachse durchgehend innerhalb des Prothesenkörpers verläuft. Durch die genannte Massenverteilung ist die Prothese in allen Schnittebenen optimal an die Form des Oberschenkelschafts angepaßt und bietet einen guten Sitz.

Die mediale Längskante der Prothese weist in ihrem proximalen Abschnitt eine konkave Krümmung auf. Die Krümmung ist stärker als bei herkömmlichen Prothesen, und der Krümmungsradius beträgt vorzugsweise weniger als 10 cm oder weniger als 8 cm, besonders bevorzugt etwa 4 bis 6 cm. Mit diesem längskonkaven Anteil legt sich die Prothese exakt der kraftaufnehmenden Struktur des medialen Femuranteils an. Die Massenverteilung, d.h. die Dicke der Prothese im Längsschnitt, ist entlang dieser konkaven Oberfläche vorzugsweise so ausgebildet, daß die Hauptmasse proximal, d.h. kopfnahe verteilt ist und von hier aus vorzugsweise kontinuierlich abnimmt bis zur Spitze. Dies beruht darauf, daß Versuche gezeigt haben, daß die Krafteinleitung proximal konzentriert ist, so daß in diesem Bereich auch die Prothesenmasse konzentriert sein muß. Der gesamte Massenschwerpunkt der Prothese liegt vorzugsweise innerhalb des Knochens, um unerwünschte Kippbewegungen und Drehmomente zu verhindern. Besonders bevorzugt liegt der Massenschwerpunkt im proximalen Prothesenabschnitt im medialen und dorsalen Teil der Prothese, er kann gegebenenfalls aber auch mittig zwischen dorsal und ventral liegen. Aufgrund der Massenabnahme nimmt auch die Steifigkeit der Prothese von proximal nach distal ab.

Untersuchungen haben gezeigt, daß in der "heel strike"-Phase, d.h. beim Aufsetzen der Ferse, ein hohes Retrotorsionsmoment auftritt, d.h. daß der Kopf nach hinten gedreht wird, wogegen während der Schwingungsphase lediglich ein geringeres Antetorsionsmoment auftritt. Um eine hohe Rotationsstabilität zu erreichen, müssen diese Momente durch die Ausladungen oder "Arme" der im Querschnitt hufeisenförmigen Prothese aufgefangen werden. Besondere Aufmerksamkeit ist der Ausladung der Hohlrinne im proximalen Bereich zu schenken. Der Stiel der Prothese kann hier flügelartige Ausladungen aufweisen, wodurch eine sehr hohe Rotationsstabilität erreicht wird. Die Flügel können bei Rechts-Links-Prothesen auch asymmetrisch ausgebildet sein. In diesem Falle wird vorzugsweise der dorsale Flügel oder Arm verbreitert und gegebenenfalls auch verlängert, da die Hauptrotationskomponente, wie vorstehend erläutert, eine Retrotorsion der Prothese darstellt und zwar beginnend beim Aufsetzen der Ferse bis zur Standbeinphase. Durch die Verkürzung des ventralen Arms kann außerdem eine Materialersparnis ohne Stabilitätsverlust erzielt werden.

Durch verschiedene Variationen in der Dicke des Stiels kann im Rahmen eines Biopsieprogrammes die in Bezug auf die Deformation der Prothese günstigste und stabilste Version bestimmt werden. Dabei wird zunächst eine beispielsweise zylinderförmige Knochenbiopsie entnommen und die individuelle Deformierbarkeit des Knochens gemessen. Danach wird mittels eines kalibrierbaren Röntgenkontrastverfahrens die Knochendichte bestimmt. Aus der individuellen Deformierbarkeit und der Knochendichte wird die gesamte Deformierbarkeit des Knochens bestimmt. Bei niedriger Deformierbarkeit kann gegebenenfalls eine zementfreie Prothese verwendet werden. Bei hoher Deformierbarkeit ist eine zementierte Prothese bevorzugt.

An Stellen hoher Krafteinleitung, also insbesondere proximal, muß die Prothese eine höhere Wandstärke aufweisen und auch der Knochenzement muß möglichst dick sein, um eine gleichmäßige Krafteinleitung ohne unerwünschte Deformation des Knochens zu ermöglichen.

Die Spitze der Prothese ist so gestaltet, daß sie vor allem nach ventral, aber auch nach lateral breite Auflageflächen bietet. An keiner Stelle sind Kanten oder Grate ausgebildet, die Anlaß zu Streßkonzentrationen und damit zu Streßfrakturen des Zementköchers geben könnten.

In einer einfachen Ausführungsform weist die Prothese eine konusförmige Aufnahme für einen beliebigen Kugelkopf auf, wie er beispielsweise in Stahl oder Keramik im Handel ist. Die Variabilität des diese Ausführungsform kennzeichenden medialen Aufbaues ist auch bei verschiedenen individuellen Femora gering, so daß die gängigen morphologischen Varianten der Hüftgelenke mit drei bis vier Größen abgedeckt werden können. Die Zahl läßt sich noch weiter verringern, wenn eine variable Aufnahme am Schenkelhals vorgesehen ist, die in der Frontalebene verstellbar ist. Gegebenenfalls ist auch eine zusätzliche Verstellbarkeit in der Sagittalebene und/oder in der Höhe vorgesehen.

Die Bestimmung der Prothesengrößen orientiert sich vorzugsweise an der Distanz der Femurschaftachse in der Ebene des Trochanter minors zur tragenden Struktur medial am Femur.

Die Grundachse oder Säule der Konstruktion stellt die Femurschaftachse dar. Der Schaft oder Stiel der Prothese ist so konstruiert, daß er entlang dieser Achse geführt wird und stabil fixiert sitzt. Dies kann einerseits durch die Länge des Stieles und andererseits auch durch das vorstehend erläuterte Profil des Querschnittes erreicht werden.

Die Prothese kann auch so konstruiert werden, daß mehrere Komponenten übereinander an einer hohlen oder rinnenförmigen Achse aufgereiht werden, wodurch ein Modularsystem geschaffen werden kann.

Die Länge der Prothese kann variabel gestaltet werden und beträgt in der Regel zwischen 13 und 22 cm. Die Länge ist besonders variabel zu gestalten, wenn die Prothese als Modularsystem aufgebaut ist.

Das modulare Baukastensystem, bei dem ein halboffenes Rohr bzw. eine Hohlrinne als Achse die modularen Komponenten aufnimmt, bietet für Reoperationen entscheidende Vorteile, da die Komponenten einzeln ausgetauscht werden können.

Für die zementfreie Implantation kann die Prothese auch vorgespannt eingesetzt werden. Dies ist besonders bevorzugt, wenn gewährleistet werden soll, daß poröse oder spongiosaartig strukturierte Oberflächen knöchern integriert werden sollen und Relativbewegungen von Oberflächen vermieden werden müssen. Zu diesem Zweck wird die Prothese über den Kopf verhakt und distal vorgespannt oder es wird umgekehrt verfahren. Es zeigt sich zwar, daß diese Vorspannung im Verlauf der folgenden drei Monate zum größten Teil abgebaut wird, in dieser Zeit kann jedoch eine knöcherne Überbrückung erfolgen.

Die Prothese kann aus einer gängigen Co-Cr-Mo-Legierung gegossen oder geschmiedet werden. Sie kann auch aus Titan oder einer Ti-Legierung gefertigt werden. Es ist auch möglich, daß die Prothese zur Erhöhung der Oberfläche nach Art der Spongiosametalle durchgehend porös ist oder daß sie mit einer porösen Beschichtung oder einer Keramik-, Bioglas- oder Apatitbeschichtung hergestellt wird.

Der Stiel der Prothese kann vorzugsweise auch aus geschichteten Gittern geformt werden, die punktuell oder über Tragrippen verschweißt werden.

Die Erfindung wird nachstehend anhand der Zeichnung näher erläutert. Es zeigen:
- Figur 1: eine Ausführungsform der erfindungsgemäßen, im Femur implantierten Prothese, wodurch zur näheren Erläuterung der Querschnitt der Prothese im proximalen Abschnitt schematisch eingezeichnet ist,
- Fig. 1a bis 1c: typische Prothesenquerschnitte, z.B. im proximalen Bereich des Stiels,
- Figur 2: eine weitere Ausführungsform der erfindungsgemäßen Prothese mit einer Halbschale, wobei ebenfalls verschiedene Querschnittsflächen schematisch eingezeichnet sind,
- Figur 3: eine weitere Ausführungsform der erfindungsgemäßen Prothese mit schematisch eingezeichneten Querschnittsflächen,
- Fig. 4a bis 4b: verschiedene Ausführungsformen der Verschiebbarkeit des Steckkonus,
- Figur 5: eine erfindungsgemäße, als Modularsystem aufgebaute Prothese, und
- Fig. 6a bis 6d: verschiedene Phasen bei der Verankerung der erfindungsgemäßen Prothese.

In den Zeichnungen sind die Prothesen, falls nicht anders angegeben, jeweils als (im implantierten Zustand) Vorderansicht gezeigt.

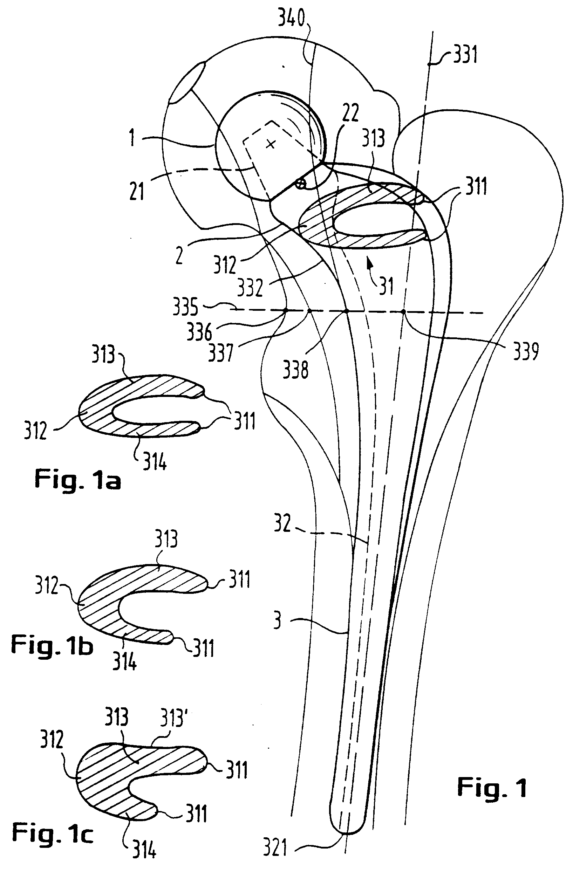

Die schematisch im Oberschenkelknochen dargestellte Prothese gemäß Figur 1 weist einen aufsteckbaren Kopf 1 aus Keramik oder Stahl auf, der auf dem konusförmig ausgebildeten Teil 21 des Halses 2 der Prothese aufsitzt, wobei der Winkel zur Schaftachse zwischen 120 und 150° beträgt. Dieser Winkel ist über eine Achse 22 einstellbar und fest zu fixieren, oder er ist in der Konstruktion vorgegeben, in der Regel als 140°.

Der Halsteil 2 ist fest mit dem Stiel 3 verschweißt. Der Stiel 3 weist in Längsrichtung medial eine konkave Krümmung 332 auf, die sich den auf das tragende Dach gerichteten tragenden Strukturen medial im Femur anpaßt. Diese Strukturen lassen sich beispielsweise durch Röntgenaufnahmen sichtbar machen. Nach dem Wolff'schen Gesetz richtet sich der Knochen nach der Beanspruchung aus und paßt sich dieser an. Aus der Richtung der tragenden Strukturen bzw. Trabekel läßt sich somit die Richtung der Beanspruchung bzw. die Richtung der Krafteinleitung ermitteln. Dem trägt die Ausbildung der konkaven Krümmung 332 Rechnung.

Im Längsschnitt, der in Figur 1 schematisch dargestellt ist, siehe die gestrichelte Linie 32, wobei der Prothesenteil medial der Linie 32 massiv ausgebildet ist, weist die Prothese einen massiven Halsteil und eine nach distal abnehmende Schichtdicke bzw. Wandstärke auf. Die Spitze 321 der Prothese ist rund gestaltet.

Die Größe der Prothese wird anhand des Röntgenbildes mit Hilfe der vorzugsweise durch den Einschlagpunkt der Prothese verlaufenden Schaftachse 331 und der gestrichelt eingezeichneten Trochanter-minor-Schnittebene 335 bestimmt. Die Trochanter-minor-Schnittebene 335 ergibt sich aus der horizontalen Beanspruchungsebene im Kniegelenk und ist in der Regel um etwa 81° zur Schaftachse geneigt. Der Punkt 339 stellt den Schnitt der Trochanter-minor-Schnittebene 335 mit der Schaftachse 331 dar, der Punkt 338 den Schnitt der Trochanter minor-Schnittebene 335 mit der Linie 340 der Krafteinleitung bzw. der Projektionslinie der tragenden Struktur, der Punkt 337 den Schnitt der Trochanter-minor-Schnittebene mit der inneren (endostalen) Knochenrinde bzw. dem Markhöhlenrand, und der Punkt 336 den Schnitt der Trochanter-minor-Schnittebene 335 mit dem Knochen an der Einsenkung am Übergang zum Trochanter minor, wo der Muskelansatz liegt. Die zu verwendende Prothesengröße ergibt sich entweder direkt aus der Strecke zwischen den Punkten 339 und 338 oder wird indirekt aus der Distanz zwischen den Punkten 339 und 337 bzw. den Punkten 339 und 336 bestimmt.

Der Querschnitt des Stieles, der schematisch beispielsweise als 31 eingezeichnet ist, ist vorzugsweise ellipsenförmig, parabelförmig, U-förmig oder hufeisenförmig und kann gegebenenfalls auch ein (dreiblättriges) Kleeblattprofil aufweisen. Die Enden 311 der Arme des U bzw. des Hufeisens sind stumpf abgerundet. Die Massenverteilung bzw. die Wandstärke des Querschnitts ist vorzugsweise exzentrisch, wobei medial (bei 312) und dorsal (bei 313) Verdickungen vorgesehen sind, da dort hauptsächlich die Kraftübertragung erfolgt.

Bei dem in Figur 1a dargestellten typischen Prothesenquerschnitt im proximalen Bereich sind der dorsale Arm oder Flügel 313 und der ventrale Arm oder Flügel 314 des Prothesenquerschnitts im wesentlichen gleich lang ausgebildet, während bei der Ausführungsform gemäß Figur 1b der ventrale Arm oder Flügel 314 kürzer als der dorsale Arm oder Flügel 313 ist. Dies führt zu einer weiteren Materialersparnis ohne wesentliche Stabilitätseinbußen, da die Krafteinleitung vorwiegend im medialen und dorsalen Bereich erfolgt.

Bei der Ausführungsform des Querschnitts gemäß Fig. 1c weist der dorsale Arm 313 an seiner Außenseite eine konkave Einbuchtung 313' auf. Hierdurch wird die Rotationsstabilität weiter verbessert.

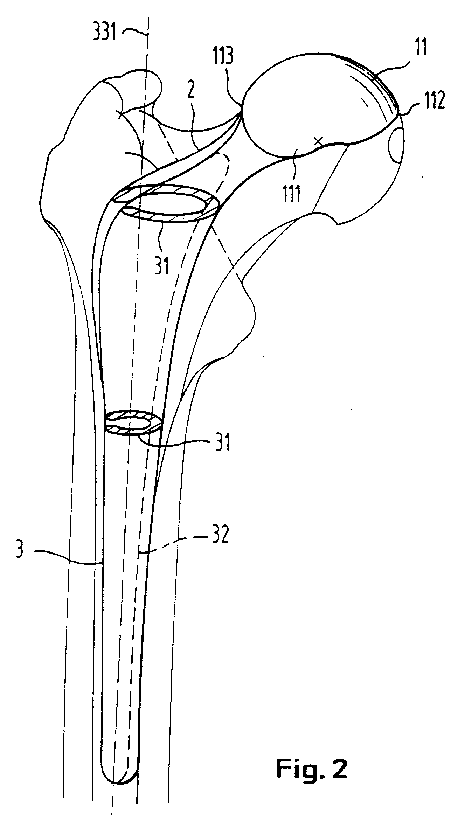

Die in Figur 2 dargestellte Rechtsprothese ist, soweit nicht anders angegeben, ähnlich ausgebildet, wie die Linksprothese gemäß Figur 1.

Bei der in Figur 2 dargestellten Prothese ist dem mit dem Stiel 3 fest verschweißten Hals 2 entweder fest oder gelenkig eine Halbschale 11 aufgesetzt. Diese ist sphärisch und weist ventral und dorsal ausgezogene Oberflächen 111 auf.

Die Kappe endet medial bei 112 vorzugsweise mit einem eingezogenen stumpfen Rand in Höhe der fovea capitis, wo ein Band ansetzt. An ihrem massiven lateralen Ende 113 geht die Schale in den stabilen, vorzugsweise massiven Hals über. Die Schale wird zum medialen Rand hin dünner. Die Schale kann gegebenenfalls als Teil über eine (in Fig. 2 nicht dargestellte) Schiene aufgesetzt und damit in der frontalen Ebene exakt eingestellt werden. Sie kann gegebenenfalls auch mit einem Konus verstellbar in den Halsteil 2 der Prothese eingesteckt werden.

Der Kopf aus Keramik oder Stahl kann auch in fester Verbindung auf eine feste Basis des Halsteils 2 aufgebracht werden.

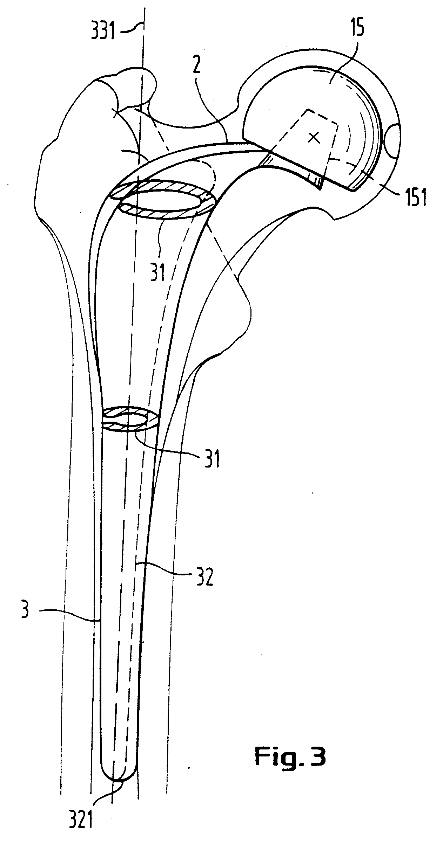

Wie bei der Prothese gemäß Figur 3 dargestellt, kann der Kopf 15 auch auf einer Konus 151 des in proximalen Bereich massiven Halsteils 2 aufgesteckt sein.

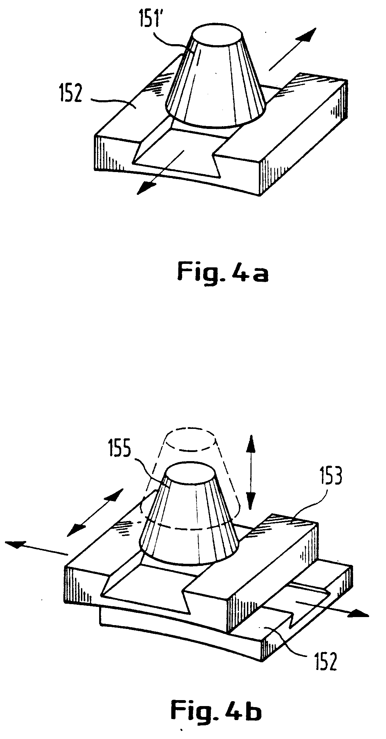

In den Figuren 4a und 4b ist dargestellt, daß das Halsteil so ausgebildet sein kann, daß der Kopf in ein, zwei oder drei Ebenen verstellbar und exakt einstellbar ist.

Gemäß Figur 4a ist ein Steckkonus 151', auf den der Kopf aufgesteckt wird, auf einer Schiene 152, welche mit dem massiven Halsteil der Prothese fest verschweißt ist, in der Frontalebene (parallel zur Stirn) in Richtung der Pfeile verschiebbar. Die Schiene kann beispielsweise als Schwalbenschwanzführung ausgebildet sein.

Anstelle der Ausführungsform gemäß Figur 4a kann die Schiene auch gekrümmt sein, und der Steckkonus kann ferner in der Sagittalebene (senkrecht zur Stirn) kippbar sein, vorzugsweise um 5 bis 10° und ebenfalls nach dorsal.

Gemäß Figur 4b ist der Steckkonus 155 für einen Kopf oder eine Schale mittels zweier zueinander senkrechter Schienen 152 und 153 sowohl in der Frontalebene als auch in der Sagittalebene verschiebbar bzw. kippbar und ist außerdem in Vertikalrichtung beweglich (vgl. die gestrichelt eingezeichnete Stellung). Der Konus ist somit, wie durch die Pfeile angedeutet, in drei Ebenen verstellbar und beispielsweise mittels Schrauben in jeder Position fest fixierbar.

Neben den in den Figuren 4a und 4b lediglich schematisch gezeigten Ausführungsformen können auch noch andere Mittel gewählt werden, um den Kopfteil der Prothese in ein, zwei oder drei Richtungen bzw. Ebenen verstellbar zu machen, um ein perfektes Alignment zu gewährleisten.

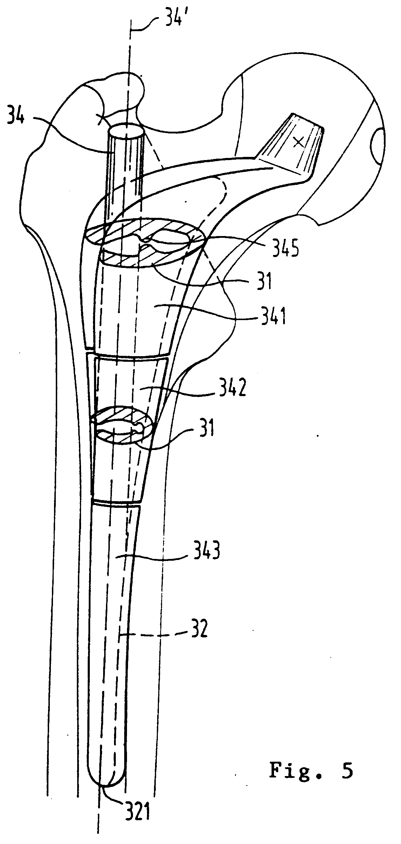

Figur 5 zeigt schematisch eine als Modularsystem aufgebaute Prothese. Diese besteht aus einer lateral, d.h. seitlichkörperfern in der Markhöhlenachse 34' angeordneten Achse 34 und aufsteckbaren medialen Komponenten 341, 342 und 343. Wie anhand der Komponente 341 dargestellt, weisen die Komponenten eine die Achse 34 umfassende Führung 345 auf, deren Form an die Achse 34 angepaßt ist, und sind im übrigen ebenfalls als im Querschnitt hufeisenförmige Hohlkörper ausgebildet (vgl. die Querschnitte 31).

### Beispiel

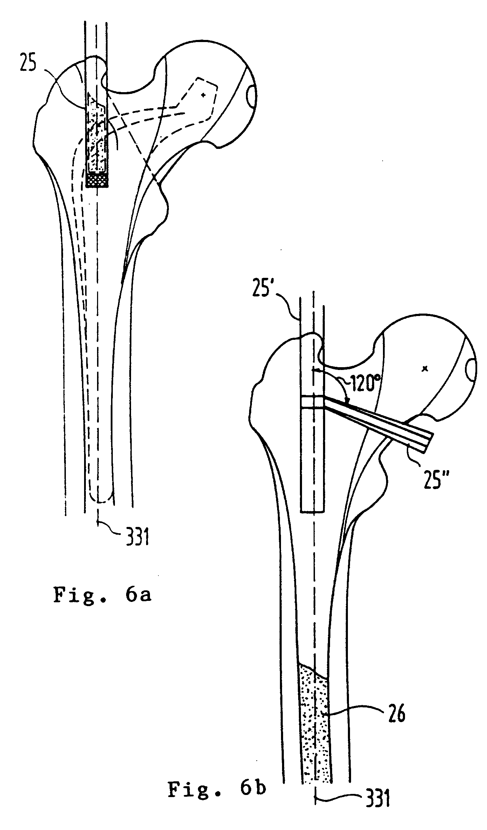

Die Figuren 6a bis 6d zeigen verschiedene Phasen des Einsetzens einer erfindungsgemäßen Prothese.

Bei einem Patienten mit fortgeschritten arthrotisch verändertem Hüftgelenk soll ein künstliches Gelenk eingesetzt werden. Nach Anfertigen von zwei Röntgenbildern des Hüftgelenkes (anterior-posterior und axial) mit eingeblendetem Maßstab wird ein endoprothetischer Ersatz geplant. Nach Auswahl der entsprechenden Prothese und den üblichen Operationsvorbereitungen wird der Patient operiert und das erkrankte Hüftgelenk dargestellt. Nach Eröffnung und Abtragen der Gelenkkapsel wird das runde Kopfband durchtrennt und der Hüftkopf luxiert. Nach Entfernen der restlichen Kapsel wird die Markhöhle in ihrer geraden Verlängerung zwischen Trochanter major (großer Rollhügel) und dem Schenkelhals mit einer Diamanthohlschleife, beispielsweise gemäß EP-A-99 371, in der Weise eröffnet, daß nach dem Naßschleifen eines ca. 14 mm messenden Ringdefektes mit einer Breite von ca. 50 mm der kortiko-spongiöse Knochenzylinder mit einem speziellen Extraktor, beispielsweise ebenfalls gemäß EP-A-99 371, entnommen wird und im Anschluß daran mit einer langen Zylinderhohlschleife im Naßschleifverfahren die Markhöhle vorbereitet wird (siehe Figur 6a mit der Zylinderhohlschleife 25).

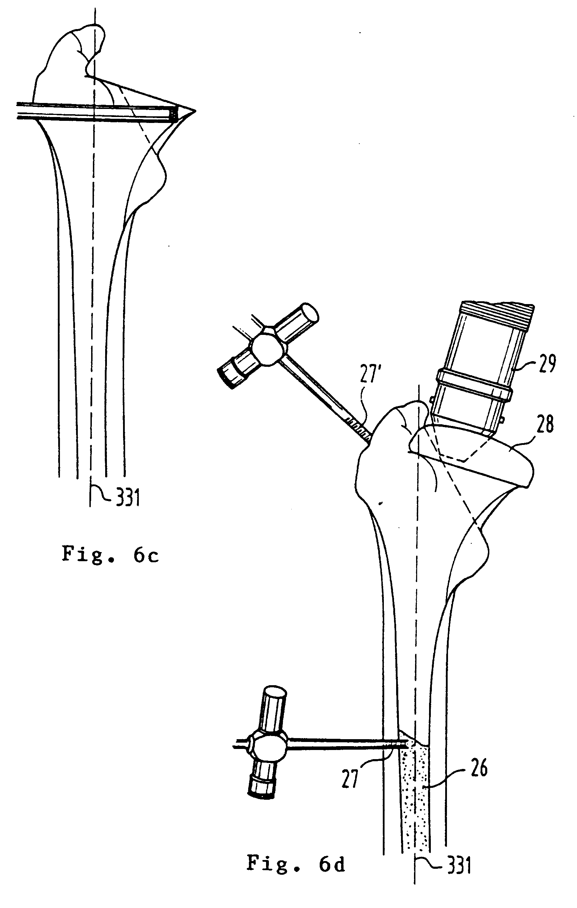

Nach der Drucklavage des knöchernen Lagers wird ein Führungsinstrument 25' eingesetzt, welches mittels einer Schablone 25'' die exakte Schnittebene für das Absetzen des Schenkelhalskopfes vorgibt (siehe Figur 6b). Distal ist die Markhöhle mit einem Plug 26 abgeschlossen. Nach der Resektion des Kopfes wird mit einer im Durchmesser 10 mm messenden Diamanthohlschleife in der Horizontalebene des Calcar femoris (medialer Schnittrand des Schenkelhalses) ein Querstollen in frontaler Richtung von lateral nach medial gefräst (siehe Figur 6c). Der Defekt wird nach außen mit einem verblockenden, jedoch kürzeren Knochenzylinder wieder verschlossen.

Nunmehr wird der zur Eröffnung der Markhöhle entnommene Zylinder gewaschen und wieder eingesetzt und mit einem Kunststoffstössel nach distal verschoben. 2 cm unterhalb der geplanten Prothesenspitze wird die Markhöhle nach Stichincision und Vorschieben eines Troikas anterolateral eröffnet und eine selbstschneidende Kanüle 27, beispielsweise gemäß EP-A-305 417, in die Kompakta eingeschraubt. Durch die Kanüle wird ein Kirschnerdraht eingeschoben. Der Knochenzylinder wird von proximal soweit vorgeschoben, bis der Kirschnerdraht wackelt. Dieser wird daraufhin zurückgezogen und der Plug noch 2 cm vorgeschoben und die Kanüle drei Umdrehungen weiter vorgedreht. Das in dieser Weise vorbereitete Knochenbett nimmt zunächst die Probeprothese auf, mit der der Prothesensitz getestet wird. Sobald die Probeprothese ideal sitzt und sich der medialen Spongiosa großflächig anlegt, wird sie wieder herausgenommen. Nunmehr wird in die dorsale Hälfte des Trochantermassives ebenfalls eine Drainagekanüle 27' eingeschraubt und beide Kanülen werden an eine Vakuumpumpe angeschlossen, welche jedoch noch nicht eingeschaltet ist. Der Schlauch zur proximalen Kanüle bleibt zunächst abgeklemmt.

Die Markhöhle wird nun mit einem Silikonaufsatz 28 versiegelt, die Knochenzementspritze 29 wird aufgesetzt und die Vakuumpumpe eingestellt (siehe Figur 6d). Der Zement wird dadurch gleichmäßig in ein bluttrockenes Bett eingesaugt und füllt die Spongiosawaben im tragenden Bereich aus. Nach dem Absetzen der Zementspritze wird die Prothese langsam eingeschoben, bis sie in der vorher bestimmten Weise sitzt. Nach Aushärten des kaltpolymerisierenden Knochenzementes wird die Pfanne eingesetzt und die Operation wird nach Reposition der Femurkomponente und nach Verschluß der Wunde in Schichten beendet.

## Patentansprüche

1. Hüftgelenksprothese mit einem in eine Femurmarkhöhle implantierbaren Stiel (3), der zumindest über einen wesentlichen Teil seiner Länge aus einem Hohlkörper besteht, dadurch gekennzeichnet, daß der Hohlkörper im Querschnitt etwa hufeisenförmig oder U-förmig und an der lateralen Seite der Prothese offen ist.

2. Prothese nach Anspruch 1, dadurch gekennzeichnet, daß der ventrale Arm (314) des Prothesenquerschnitts kürzer ist als der dorsale Arm (313).

3. Prothese nach Anspruch 1 oder 2, dadurch gekennzeichnet, daß zumindest im proximalen Prothesenabschnitt die Wandstärke des Hohlkörpers dorsal und medial verdickt ist.

4. Prothese nach einem der Ansprüche 1 bis 3, dadurch gekennzeichnet, daß die Wandstärke des Hohlkörpers von proximal nach distal abnimmt.

5. Prothese nach einem der Ansprüche 1 bis 4, dadurch gekennzeichnet, daß sie aus einem Kopfteil (1) einem Halsteil (2) und dem Steil (3) besteht, wobei gegebenenfalls der Halsteil (2) als lateral offener Hohlkörper ausgebildet sind.

6. Prothese nach einem der Ansprüche 1 bis 5, die als Geradschaftprothese ausgebildet ist, die in ihrer Seitenansicht eine S-förmige Massenverteilung aufweist, wobei der Massenschwerpunkt der Prothesenquerschnitte im proximalen Prothesenabschnit dorsal und im distalen Prothesenabschnitt ventral von der Prothesenachse (331) angeordnet ist.

7. Prothese nach einem der Ansprüche 1 bis 6, mit einem Halsteil (2) und einem Kopfteil (1), wobei der Kopfteil (1) der Prothese ein-, zwei- oder dreidimensional verstellbar angeordnet ist, wobei die Verstellbarkeit in den einzelnen Richtungen vorzugsweise unabhängig voneinander ist.

8. Prothese nach einem der Ansprüche 1 bis 7, dadurch gekennzeichnet, daß die Hauptmasse der Prothese proximal ausgebildet ist und ihr Massenschwerpunkt innerhalb des Knochens liegt, in den sie implantiert wird.

9. Prothese nach einem der Ansprüche 1 bis 8, dadurch gekennzeichnet, daß der Massenschwerpunkt des Querschnitts der Prothese in ihrer Lateral-Medial-Richtung im medialen Teil und in ihrer Dorsal-Ventral-Richtung im dorsalen Teil oder mittig liegt.

10. Prothese nach einem der Ansprüche 1 bis 9, dadurch gekennzeichnet, daß die Steifigkeit der Prothese von proximal nach distal abnimmt.

11. Prothese nach einem der Ansprüche 1 bis 10, dadurch gekennzeichnet, daß der Querschnitt der Prothese zumindest in ihrem proximalen Abschnitt im dorsalen Bereich eine konkave Wölbung aufweist.

12. Prothese nach einem der Ansprüche 1 bis 11, dadurch gekennzeichnet, daß sie modular aus mehreren Komponenten (341,342,343) aufgebaut ist.

13. Prothese nach Anspruch 12, gekennzeichnet durch eine Achse (34), auf die die einzelnen Komponenten (341,342,343) mittels einer Führung aufsteckbar sind, wobei die Komponenten vorzugsweise mindestens teilweise als seitlich offene Hohlkörper ausgebildet sind.

14. Verwendung der Prothese nach einem der Ansprüche 1 bis 13 bei Hüftgelenksoperationen.

## Claims

1. A hip joint prosthesis having a stem (3) that is implantable into a femoral medullary cavity and consists at least along an essential part of its length of a hollow body, characterized in that the hollow body has an approximately horseshoe-shaped or U-shaped cross-section and is open at the lateral side of the prosthesis.

2. The prosthesis according to claim 1, characterized in that the ventral arm (314) of the cross-section of the prosthesis is shorter than the dorsal arm (313).

3. The prosthesis according to claim 1 or 2, characterized in that at least in the proximal section of the prosthesis the wall thickness of the hollow body is dorsally and medially enlarged.

4. The prosthesis according to any of claims 1 to 3, characterized in that the wall thickness of the hollow body decreases from the proximal region to the distal region.

5. The prosthesis according to any of claims 1 to 4, characterized in that it consists of a head portion (1), a neck portion (2) and the stem (3), wherein the neck portion (2) is optionally a laterally open hollow body.

6. The prosthesis according to any of claims 1 to 5, which is designed as a straight-stemmed prosthesis whose side view comprises an S-shaped mass distribution, the centre of the mass of the cross-sections of the prosthesis being arranged dorsally to the prosthesis axis (331) in the proximal section of the prosthesis, and ventrally to the prosthesis axis (331) in the distal section of the prosthesis.

7. The prosthesis according to any of claims 1 to 6, comprising a neck portion (2) and a head portion (1), wherein the head portion (1) of the prosthesis is arranged adjustably in one, two or three dimensions, the individual directions preferably being adjustable independently of each other.

8. The prosthesis according to any of claims 1 to 7, characterized in that the main mass of the prosthesis is in the proximal portion and the centre of its mass lies within the bone into which it is being implanted.

9. The prosthesis according to any of claims 1 to 8, characterized in that the centre of the mass of the cross-section of the prosthesis lies in the medial portion in its lateral-medial direction, and in the dorsal portion or in the centre in its dorsal-ventral direction.

10. The prosthesis according to any of claims 1 to 9, characterized in that the stiffness of the prosthesis decreases from the proximal region to the distal region.

11. The prosthesis according to any of claims 1 to 10, characterized in that the cross-section of the prosthesis comprises a concave arch at least in its proximal section in the dorsal region.

12. The prosthesis according to any of claims 1 to 11, characterized in that it is of modular construction and comprises a plurality of components (341, 342, 343).

13. The prosthesis according to claim 12, characterized by an axis (34) on which the individual components (341, 342, 343) can be detachably mounted via a guide, wherein the components are preferably, at least partially, laterally open hollow bodies.

14. The use of the prosthesis according to any of claims 1 to 13 in hip joint surgery.

## Revendications

1. Prothèse de la hanche comprenant une tige (3) qui est implantable dans une cavité médullaire de fémur et qui est constituée d'un corps creux au moins sur une partie importante de sa longueur, caractérisée en ce que le corps creux présente en section transversale approximativement une forme de fer à cheval ou de U et est ouvert sur le côté latéral de la prothèse.

2. Prothèse suivant la revendication 1, caractérisée en ce que la branche ventrale (314) de la section transversale de prothèse est plus courte que la branche dorsale (313).

3. Prothèse suivant l'une des revendications 1 et 2, caractérisée en ce qu'au moins dans le tronçon proximale de la prothèse l'épaisseur de paroi du corps creux est épaissie dorsalement et médialement.

4. Prothèse suivant l'une des revendications 1 à 3, caractérisée en ce que l'épaisseur de paroi du corps creux diminue de l'extrémité proximale à la distale.

5. Prothèse suivant l'une des revendications 1 à 4, caractérisée en ce qu'elle est constituée d'une partie de tête (1), d'une partie de col (2) et de la tige (3), la partie de col (2) étant éventuellement réalisée sous la forme d'un corps creux latéralement ouvert.

6. Prothèse suivant l'une des revendications 1 à 5, qui est réalisée sous la forme d'une prothèse à hampe droite, qui présente, dans sa vue latérale, une répartition de masse en forme de S, le centre de gravité des sections transversales de prothèse étant disposé, dans le tronçon proximal de la prothèse, dorsalement par rapport à l'axe de prothèse (331) et, dans le tronçon distal de la prothèse, ventralement.

7. Prothèse suivant l'une des revendications 1 à 6, comprenant une partie de col (2) et une partie de tête (1) dans laquelle la partie de tête (1) de la prothèse est agencé de manière réglable dans une, deux ou trois dimensions, les capacités de réglage dans les différentes directions étant de préférence indépendantes l'une de l'autre.

8. Prothèse suivant l'une des revendications 1 à 7, caractérisée en ce que la masse principale de la prothèse est prévue en position proximale et en ce que son centre de gravité se trouve à l'intérieur de l'os dans lequel elle est implantée.

9. Prothèse suivant l'une des revendications 1 à 8, caractérisée en ce que le centre de gravité de la section transversale de la prothèse se trouve, dans sa direction latérale-médiale, dans une partie médiale et, dans sa direction dorsale-ventrale, dans une partie dorsale ou centralement.

10. Prothèse suivant l'une des revendications 1 à 9, caractérisée en ce que la rigidité de la prothèse diminue de l'extrémité proximale à la distale.

11. Prothèse suivant l'une des revendications 1 à 10, caractérisée en ce que la section transversale de la prothèse présente un cintrage concave dans la zone dorsale, au moins dans son tronçon proximal.

12. Prothèse suivant l'une des revendications 1 à 11, caractérisée en ce qu'elle est constituée de manière modulaire à partir de plusieurs composants (341, 342, 343).

13. Prothèse suivant la revendication 12, caractérisée par un axe (34) sur lequel les différents composants (341, 342, 343) peuvent être emmanchés au moyen d'un guidage, les composants étant de préférence réalisés au moins partiellement sous la forme de corps creux latéralement ouverts.

14. Utilisation de la prothèse suivant l'une des revendications 1 à 13, dans des opérations de l'articulation de la hanche.
